# EUROPEAN PATENT APPLICATION

(11) **EP 3 943 052 A1**
(43) Date of publication of application: **26.01.2022**
(21) Application number: 20187686.9
(22) Date of filing: 24.07.2020
(51) Int. Cl.: A61F 9/008

(54) **SYSTEM FOR IN VIVO TREATMENT OF A PATIENT'S EYE'S ENDOTHELIUM AND/OR DESCEMET'S MEMBRANE**

(71) Applicant: Klinikum der Universität München Anstalt des öffentlichen Rechts, 81377 München (DE)
(72) Inventor: KASSUMEH, Stefan, 80333 München (DE); OHLMANN, Andreas, 80801 München (DE); BIRNGRUBER, Reginald, 23564 Lübeck (DE); PRIGLINGER, Siegfried, 81247 München (DE)
(74) Representative: Vossius & Partner Patentanwälte Rechtsanwälte mbB

(57) **Abstract**

The invention relates to a system a method and a use of a laser for in vivo treatment of a patient's eye's endothelium and/or Descemet's membrane by laser therapy. The system comprises a laser unit comprising a short-pulse laser with a wavelength above 800 nm, and a fixation unit for fixing the patient's eye, wherein the laser unit is configured to set the laser focus to the endothelium and/or Descemet's membrane and wherein the laser is adapted to generate tissue effects having an accuracy of 5 µm or less in laser-beam direction in the endothelium and/or Descemet's membrane.

## Description

### Technical field

The invention relates to a system for in vivo treatment of a patient's eye's endothelium and/or Descemet's membrane by laser therapy, a method of *in vivo* treatment of a patient's eye's endothelium and/or Descemet's membrane by laser therapy and a use of a laser for *in vivo* treatment of a patient's eye's endothelium and/or Descemet's membrane.

### Background

The cornea is the transparent part of the eye visible from the outside. In addition to its protective function, the cornea contributes significantly to the optical refractive power of the eye (up to ¾ of total refractive power). The prerequisite for unrestricted vision is, in addition to the compensation of any refractive errors, the absolute transparency of all layers of the cornea. These include the corneal epithelium, the Bowman's membrane, the stroma, the Descemet's membrane and the endothelium. The latter is essential for the regulation of the corneal water balance. Damage to corneal endothelial cells, for example due to trauma, surgery or corneal endothelial diseases, can lead to a loss of function of the water pumps of these cells. The consequence is a disproportionate influx of water with consecutive swelling (edema formation) of the corneal stroma, an increase in thickness and finally clouding of the cornea. Typically, the affected patients describe an increasing sensitivity to glare as well as a gradual reduction of vision. In severe or untreated cases a total loss of vision may occur.

One of the most frequent diseases of the corneal endothelium and finally also one of the more frequent causes of the above mentioned course of the disease is the so-called Fuchs' corneal endothelial dystrophy. This disease causes deposits of extracellular matrix on the Descemet's membrane. This leads to the formation of button-like protuberances (the so-called "guttae") of this matrix, which are pushed between the endothelial cells, displace them and thus continuously contribute to their loss of function.

The current surgical technique for the treatment of Fuchs' endothelial dystrophy is only possible with corneal donor material. In this operation, a graft of a thin membrane consisting of corneal endothelium, Descemet's membrane and possibly adjacent corneal stroma is prepared from a donor cornea and, after removal of the diseased layer of endothelium and Descemet's membrane of the patient's cornea, is transferred to the inside of the patient's cornea. The removal of this layer in the patient is still executed manually. The graft is attached by an air bubble, which is inserted into the anterior chamber of the patient's eye. Because of the procedure and the use of corneal donor material, the procedure was named "Descemet's Membrane Endothelial Keratoplasty (DMEK)". A consistent postoperative supine position for the first hours to days is essential to ensure proper attachment of the transplanted membrane. Gradually, the donated endothelial cells then swell the cornea so that it clears up again and the patient's visual acuity increases significantly.

The supply of corneal transplants is currently a big problem worldwide. It is estimated that in 2012, almost 200,000 corneal transplants were performed, of which about 40% were used for the therapy of Fuchs' endothelial dystrophy. Since the provision of donor corneas is complex and costly, more than half of the world's population has no access to corneal transplants.

All in all, the microsurgically demanding operation described above is a procedure that is stressful for the patient (long postoperative supine position, in-patient stay of several days) and associated with considerable complications, which also entails considerable costs. In addition, the current therapy option (DMEK) as an intraocular procedure is associated with a not inconsiderable complication rate. The most common and most feared complication is graft detachment, in which the donor membrane detaches from the inner surface of the recipient cornea. This complication occurs in approximately 15% of cases and requires a new air injection or, as an ultima ratio, another corneal transplantation.

The approach of manually removing the Descemet's membrane also has limitations: On the one hand, it is not possible to control the exact depth of the ablation, and there is a risk of removing healthy corneal tissue (even more than just the Descemet's membrane). This can lead to a worse outcome in terms of reduced visual acuity or delayed healing. On the other hand, there is no precise incision in the lateral extension. Here too, healthy tissue is usually removed unintentionally.

Finally, the input of human donor material is necessary, since the removal of the diseased triple-layer Descemet membrane removes the foundation of the corneal endothelial cells. Due to this fact, an independent recovery of the corneal endothelium cannot be expected without further influences.

### Summary

An object of the invention may be seen in increasing the success rate and/or reduce the risk of complications in therapies of the inner portion of the cornea, in particular therapies of the Fuchs' dystrophy. A further object of the invention may be seen in providing therapies for diseases of the inner portions of the cornea, e.g., the Fuchs' dystrophy, which do not rely on donor material. A further object of the invention may be seen in providing non-invasive therapies of diseases of the inner portions of the cornea, e.g., the Fuchs' dystrophy. A further object of the invention may be seen in allowing to remove inner portions of the cornea, in particular the first two inward layers of the Descemet's membrane and to allow an improved migration of endothelial cells, e.g., for treatment of the Fuchs' corneal endothelial dystrophy. Further objects of the invention may be seen in providing a system for achieving the above objects.

These and further objects are achieved by the invention defined in the appended claims. Further embodiments of the invention are set out in the dependent claims, the description and the drawings.

An aspect of the invention relates to a system for in vivo treatment of a patient's eye's endothelium and/or Descemet's membrane by laser therapy. The system comprises a laser unit comprising a short-pulse laser, wherein the laser unit is configured to set the laser focus to the endothelium and/or Descemet's membrane and wherein the laser is adapted to generate tissue effects having an accuracy of 5 µm or less in laser-beam direction in the endothelium and/or Descemet's membrane. Furthermore, the system comprises a fixation unit for fixing the patient's eye.

In the following, it will be referred to different directions with respect to the laser beam used to generate tissue effects in the patient's eye. The Z direction refers to the direction along the laser beam away from the laser. The directions X and Y are perpendicular to the laser beam. During treatment of the patient's eye, the Z direction may be perpendicular to a corneal surface, preferably the posterior corneal surface. However, the Z direction does not always have to be perpendicular to the corneal surface. For example, in order to remove a guttae, it may be preferable to adjust the Z direction with respect to the corneal surface.

Furthermore, the term "inside" or "inner" or the like refer to structures that are closer to the centre of the patient's eye and "outside" or "outer" to structures that are further away from the centre of the patient's eye. Thus, the term "inside" or "inner" may have the same meaning as "proximal" and the term "outside" or "outer" may have the same meaning as "distal".

With said laser unit, the patient's endothelium and/or Descemet's membrane can be treated externally. For example, the diseased layers can be removed externally, i.e. without any kind of active involvement in the interior of the eye. Moreover, the diseased layers can be removed in such a way that no donor material is used. Rather, regeneration of the corneal endothelium may be achieved solely by preserving the basement membrane of the Descemet's membrane, which serves as a supporting framework for the migrating endothelial cells. In order to support the regeneration of the corneal endothelium, kinase inhibitors by be administered, e.g., as eyedrops (e.g., Ripasudil). Said treatment may also be referred to as descemetorhexis. This makes the treatment more accessible worldwide, may save costs, since it could theoretically be performed in an outpatient setting and no donor material is required, and in addition, there is no risk of transplant detachment or even rejection. Moreover, the removed membrane can be left in the anterior chamber of the eye or, in individual cases, rinsed out of it by a few minutes' operation. This reduces the risk of complications, shortens the operating time and once again takes a step towards the outpatient setting. With the aid of the system disclosed herein, the descemetorhexis is performed with high precision and in a more controlled manner compared to the manual procedure. As a result, there is no "tearing" of the Descemet membrane, but a much smoother cutting action. This incision inevitably results in finer edges of the remaining corneal structures, which significantly reduces the occurrence of postoperative spherical aberrations, i.e. imaging errors that can negatively affect visual acuity.

Furthermore, the laser is adapted to be used to generate tissue effects on the patient's cornea in vivo, i.e. on the living patient and not only on external donor material.

In addition, the system disclosed herein could be used in the therapy of corneal opacities or scars of other genesis (e.g. traumatic, after infections or operations) by targeted cutting of these tissue portions. This intervention can either be performed non-refractively, i.e. without altering the refractive properties of the eye, or it can be performed as a combined therapeutic-refractive intervention.

The laser may be configured to generate different kinds of tissue effects in the patient's endothelium and/or Descemet's membrane. For example, the laser may execute an incision or cut in the endothelium and/or Descemet's membrane, e.g., in order to remove a diseased portion of the endothelium and/or Descemet's membrane. Due to the laser's accuracy of 5 µm or less, the incision may be executed while preserving the basement membrane of the Descemet's membrane. Furthermore, the tissue effects generated by the laser may include a cut that is parallel to the posterior corneal surface (fixed Z-axis) in a preoperatively determined depth to remove a circular, elliptical or custom-shaped tissue membrane. Furthermore, those tissue effects may include customized cuts that specifically target diseased corneal tissue, which can be automatically depicted or individually selected by the corneal surgeon.

The laser's accuracy of 5 µm may be understood to refer to the size of the laser's focus point in laser beam direction, i.e. in Z-direction. Preferably, the size of the laser's focus point in X-Y-direction may be 5 µm or less, e.g., to allow precise removal of diseased tissue.

Preferably, the laser is configured to adjust the focus point of the laser beam with a precision of 5 µm or less at least in the laser beam or Z-direction, more preferably in every direction and/or at a distance of more than 200 µm, even more preferably a distance of more than 300 µm, from the anterior corneal surface, i.e. in the patient's endothelium or Descemet's membrane.

Preferably, the laser unit or the aiming unit described herein is configured to move the focus point of the laser beam with a precision of 5 µm or less at least in the laser beam or Z-direction, more preferably in every direction and/or at a distance of more than 200 µm, even more preferably a distance of more than 300 µm, from the anterior corneal surface, in the patient's endothelium or Descemet's membrane.

The term focus point of the laser may refer to a focused area of the laser's laser beam. The focus point may have a cylindrical shape oriented along the laser beam direction. For example, the approximate cylinder defined by the focus point may have a length of 5 µm or less and a diameter of 5 µm or less.. The focus point may also have a non-cylindrical shape. Furthermore, the focus point may be adjustable in all directions (X, Y, Z), e.g., to adjust the shape or geometry of the incision. In particular, the extension in Z direction of the focus point may be adjustable. For example, the size of the focus point in Z direction may be reduced, e.g. to 1-2 µm, e.g. by means of one or more apertures or aperture holes, e.g., in order to treat severe cases of the Fuchs' dystrophy, where the increased corneal swelling may reduce the cutting accuracy of the laser.

For example, in order to allow for an improved and precise optical tissue breakdown without larger air bubble generation, it is preferable that the size of the focus point (spot size) is equal or below 5 µm while lowering the pulse energy (preferably below 100 pJ).

In order to achieve a focus point of 5 µm or less, the laser unit preferably comprises a focusing lens mounted on a piezoelectric stage that is adjustable in X, Y and Z direction within a few centimetres above the mounted eyeball. Preferably, the distance between the focusing lens and the anterior surface of cornea is 10 cm or less. On-line alignment of the focusing lens via the piezoelectric stage is preferably provided according to a cutting pattern, which may be automatically or manually selected. Furthermore, to reduce optical aberrations and thus reduce spot size, the laser beam is preferably aligned precisely perpendicular to the human's cornea.
While the present disclosure relates to generating tissue effects in the endothelium and/or Descemet's membrane, this does not exclude that the system disclosed herein is also suited or used for treating other portions of the eye.

The fixation unit may be configured to fix the eyeball by the application of a vacuum or suction. Moreover, the fixation unit may be configured to center the cornea. The fixation unit may either be configured to maintain the curvature of the cornea or to applanate or flatten the cornea. For example, the fixation unit may be configured to flatten or applanate the cornea, such that its radius is between 30 mm and 10 mm, preferably around 30 mm, around 20 mm or around 10 mm.

Preferably, the laser has a wavelength above 800 nm. For example, the laser is a titanium-sapphire laser femtosecond laser.

Preferably, the pulse duration of the laser is shorter than one microsecond (1 µs).

Preferably, the numerical aperture (NA) of the laser is greater than 0.3
In this way, the laser may have an accuracy of 5 µm or less, in particular in Z direction. For example, an NA of 0.3 in a system with low optical aberrations may lead to a spot size below 4 µm in diameter.

Preferably, the laser has a cutting accuracy of 5 µm or less, preferably of 4 µm or less, more preferably of 3 µm or less, in every direction (X, Y and Z) in a distance of more than 200 µm or preferably a distance of more than 300 µm from the anterior corneal surface.

For example, the focus point of the laser may have a size of 5 µm or less, preferably of 4 µm or less, more preferably of 3 µm or less, in every direction (X, Y and Z) in a distance of more than 200 µm or preferably a distance of more than 300 µm from the anterior corneal surface.

Preferably, the system further comprises an imaging unit for capturing an image of the patient's eye's cornea, preferably of the posterior part of the cornea, more preferably of the Descemet's membrane and endothelium.

Preferably, the imaging unit's resolution is 5 µm or less in every dimension.

In this way, a high resolution image of the Descemet's membrane and/or endothelium may be captured. The imaging unit may thus allow to precisely identify damaged tissue of the Descemet's membrane and/or endothelium in the image. Moreover, the image may be used on-line, pre-, peri- and postoperatively to plan, perform and monitor the surgical procedure. On the one hand, this allows micrometer-precise preoperative planning and identification of the diseased structures. Moreover, the image may allow the precise execution of the intervention. In addition, a postoperative success control can be carried out on the basis of an image captured with the imaging unit. If there is no improvement or if there are remaining diseased areas in the tissue, the procedure can be planned and performed again.

The image may be a two-dimensional or three-dimensional image. Moreover, the term image may also include a plurality of images.

The imaging unit may further comprise a displaying unit for displaying the image, e.g., a built-in or external monitor, on which the image can be displayed.

The imaging unit may be configured to acquire the image in a contact or contactless manner.

The displaying unit may be configured to display an original and/or a distortion corrected and/or segmented representation of the region of interest of the patient's cornea.

The imaging unit may further comprise a planning unit as disclosed herein. Alternatively the imaging unit and the planning unit may be two separate units.

Preferably, the imaging unit comprises an optical coherence tomography (OCT) unit.

The OCT unit provides a contactless imaging method. Furthermore, it does not emit harmful radiation. Therefore, an OCT unit may be particularly advantageous.

Preferably, the OCT unit is a polarization sensitive OCT unit, a spectral domain OCT unit, a Fourier domain OCT unit and/or a swept source OCT unit.

Preferably, the axial resolution and the lateral resolution in corneal tissue of the OCT unit is 5 µm or less, preferably 4 µm or less, more preferably 3 µm or less.

The axial resolution may refer to the resolution in Z direction and the lateral resolution may refer to the resolution in X and Y direction.

Preferably, the OCT unit has a power of 20mW or less.

Preferably, the OCT unit has a central wavelength in the range of 600 nm to 1400, preferably in the range of 800 nm to 1300 nm.

A wavelength of 800 nm to 1300 nm may be particularly advantageous, as it may provide an optimal resolution in the cornea, provide an improved absorption and/or reduce the backscattering of reflected light. Moreover, with such a wavelength, a penetration depth of 1 cm or more may be achieved.

Preferably, the imaging system comprises one or more of a confocal microscopy unit, a Scheimpflug camera, a light microscope, an ultrasound biomicroscope, a specular microscopy unit

In other words, these imaging means may be provided alternatively or in addition to the OCT unit.
For example, confocal microscopy may have a higher resolution than OCT.

Preferably, the imaging system is configured to capture at least one image of the patient's cornea before and/or during the treatment of the patient's eye's endothelium and/or Descemet's membrane by laser therapy.

Preferably, the at least one image comprises at least a portion of diseased tissue of the endothelium and/or Descemet's membrane.

Preferably, the at least one image comprises at least a portion of the tissue effects generated by the laser.

Preferably, the imaging system is configured to display or visualize the at least one image.

Preferably, the system further comprises an aiming unit configured to aim the laser focus at a predetermined portion of the patient's endothelium or Descemet's membrane.

In other words, the aiming unit may assist in aiming or automatically aim the laser at the tissue to be treated. The tissue to be treated may be identified in the planning unit disclosed herein.

The aiming unit may, e.g., be a separate component or be part of the laser unit, the imaging unit, or the planning unit.

Preferably, the system further comprises a planning unit configured to assist a user in planning and/or executing the in vivo treatment of the patient's eye's endothelium and/or Descemet's membrane with the laser unit.

Preferably, the planning and/or executing is based on at least one image captured by the imaging system.

In this way, the system assists the user in the planning of the treatment. In this way, the whole treatment procedure may be more effective and the risk of complications may be reduced.

For example, the planning unit may be configured to enhance the image by adding planned and/or executed tissue effects in the image. For this purpose, the planning unit may comprise algorithms implemented in a processing unit.

Preferably, the planning unit is configured to assist a user in planning the tissue effects in the patient's cornea with the laser unit.

For example, the planning unit may comprise a displaying unit, e.g., a built-in or external monitor. Furthermore, the planning unit may comprise a user input device, e.g., a trackpad, keyboard, mouse, touchscreen or the like. The user input device may be adapted to input a distance to the posterior corneal curvature at which an incision parallel to the posterior surface of the cornea is to be carried out by the laser. For example, the distance can be input by pointing a cursor to a certain position in the image by means of the input device. Moreover, the input device may be adapted to input a three-dimensional cutting pattern in the image, wherein the three-dimensional cutting pattern is to be executed by the laser in the diseased tissue.

Preferably, the planning unit is configured to automatically detect diseased tissue and/or anatomical landmarks of the patient's cornea.

For example, the automatic detection of diseased tissue may be based on algorithms implemented in a processing unit of the planning unit. The algorithms may comprise one or more neuronal networks.

Anatomical landmarks may, e.g., be the Descemet's membrane, the endothelium or the stromal borders.

Moreover, the planning unit may be configured to automatically suggest a suggested tissue effect, e.g., a suggested incision. For example, the planning unit may include algorithms that recognize the diseased tissue and/or anatomical landmarks (e.g., the Descemet's membrane, endothelium, stromal borders). Furthermore, the planning unit may be configured to visually identify the area to be treated. For example, the planning unit may enhance the image by adding the suggested tissue effect to the image.

Preferably, the planning unit is configured to determine the tissue effects in the patient's cornea and to represent the tissue effects in the captured image. Preferably, the planning unit is configured to assist a user in three-dimensional planning of the treatment.

For example, the user may input a three-dimensional cutting pattern in the image as described above.

Preferably, the fixation unit is configured to fix the patient's eyeball, to center the cornea, and/or to flatten the cornea.

Preferably, the fixation unit comprises a mechanism for automatically tracking the eyeball during treatment.

A further aspect of the invention relates to a method of in vivo treatment of a patient's eye's endothelium and/or Descemet's membrane by laser therapy, preferably with a system according to any one of the preceding claims, the method comprising the steps of fixing and preferably flattening the patient's eye and generating tissue effects in the patient's eye's endothelium and/or Descemet's membrane with a laser.

The method may be executed with or by a system as disclosed herein, therefore, all features disclosed with reference to the system may also apply to the method.

The step of fixing and preferably flattening the patient's eye may be executed with or by a fixation unit as disclosed herein. Therefore, all features disclosed with regard to the fixation unit may also apply to the method.

The step of generating tissue effects in the patient's eye's endothelium and/or Descemet's membrane may be executed with or by a laser unit as disclosed herein. Therefore, all features disclosed with regard to the laser unit may also apply to the method.

Preferably, the method further comprises the step of capturing an image of the patient's eye's cornea.

The step of capturing an image may be executed with or by an imaging unit as disclosed herein. Therefore, all features disclosed with regard to the imaging unit may also apply to the method.

Preferably, the method may further comprise the step of displaying the image.

The step of displaying the image may be executed with or by a displaying unit as disclosed herein. Therefore, all features disclosed with regard to the displaying unit may also apply to the method.

Preferably, the method further comprises the step of planning the tissue effects in the patient's eye's endothelium and/or Descemet's membrane based on the captured image.

The step of planning the tissue effects may be executed with or by a planning unit as disclosed herein. Therefore, all features disclosed with regard to the planning unit may also apply to the method.

Preferably, the method may further comprise the step of aiming the laser at a region of interest of the endothelium and/or Descemet's membrane.

The step of aiming the laser may be executed with or by an aiming unit as disclosed herein. Therefore, all features disclosed with regard to the aiming unit may also apply to the method.

A further aspect of the invention may refer to a use of a laser for in vivo treatment of a patient's eye's endothelium and/or Descemet's membrane.

Preferably, the laser is used for in vivo treatment of Fuchs' dystrophy.

Preferably, the laser is used for removing diseased tissue of the endothelium and/or Descemet's membrane, more preferably, in a therapy where no graft tissue is provided to the inside of the patient's cornea.

### Brief Description of Drawings

Further embodiments and exemplary embodiments as well as more technical details of the invention are described with reference to the following drawings. In the drawings, the same reference numerals may be used for identical or similar elements, whereas the same or similar elements may also be denoted with different reference numerals. The invention is not limited to the exemplary embodiments.
Fig. 1A shows a possible schematic illustration of a system for in vivo treatment of a patient's eye's endothelium and/or Descemet's membrane by laser therapy.
Fig. 1B shows a possible schematic illustration of a laser of a system for in vivo treatment of a patient's eye's endothelium and/or Descemet's membrane by laser therapy.
Fig. 2 shows a schematic illustration of a patient's cornea as well as the intended cutting patterns using a system for in vivo treatment of a patient's eye's endothelium and/or Descemet's membrane by laser therapy.

### Description of Embodiments

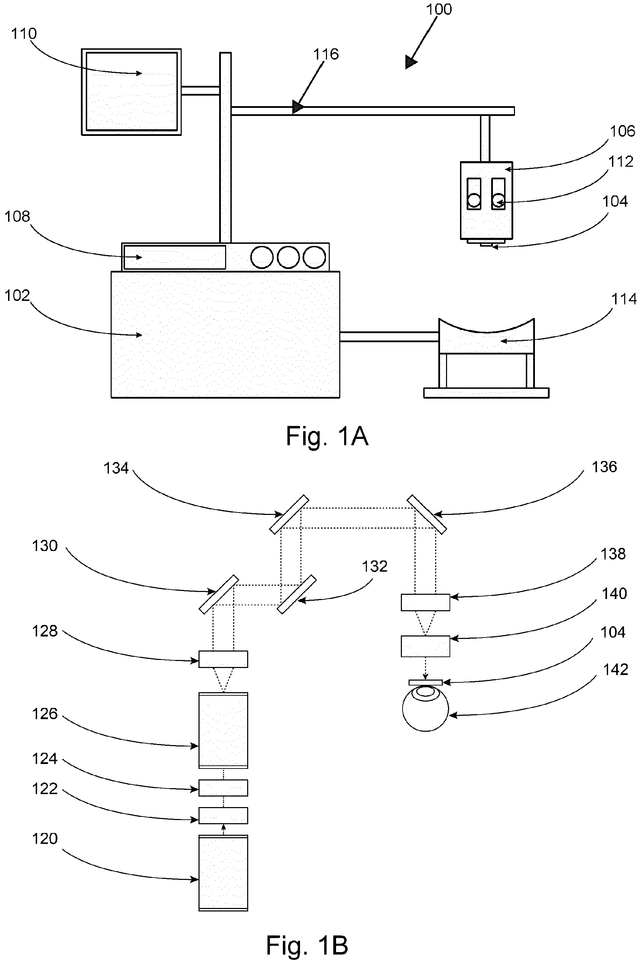

Fig. 1A shows an exemplary embodiment of a system 100 for in vivo treatment of a patient's eye's endothelium and/or Descemet's membrane.

The system 100 comprises a laser base unit 102 comprising a short-pulse laser with a wavelength above 800 nm. The laser base unit 102 is configured to generate a pulsed laser beam. Said laser beam is then guided to the patient's eye. The laser unit further comprises laser optics configured to set the laser focus and/or to generate tissue effects in the endothelium and/or Descemet's membrane with an accuracy of 5 µm or less in laser beam direction, preferably in every direction.

The system 100 further comprises a patient interface 106 connected to the laser base unit 102 via a support arm 116. The support arm 116 supports the patient interface 106 and/or guides the laser beam generated by the laser base unit 102 to the patient interface 106.

The patient interface 106 further comprises a fixation unit 104 for fixing the eye of a patient who is lying on the patient couch 114. Moreover, the fixation unit 104 is, e.g., configured to flatten the patient's cornea.

Furthermore, the patient interface 106 includes an imaging unit as described herein. Moreover, the patient interface 106 may include a slit lamp and/or oculars 112 to illuminate and/or allow a user to visually inspect a magnified region of interest of the eye. The fixation unit 104 is provided at the lower end of the patient interface 106.

Furthermore, the system 100 comprises a planning unit 108. The planning unit 108 is configured to assist a user in planning and/or executing the in vivo treatment of the patient's eye's endothelium and/or Descemet's membrane with the laser unit 102. E.g., the planning unit may receive and process the at least one image captured by the imaging system. Said at least one image may be used to assist the user in planning and/or executing the treatment. The planning unit 108 may include the features described herein.

The system 100 further comprises a displaying unit 110, e.g., a monitor. The displaying unit may be configured to display the image captured by the imaging unit. Furthermore, the displaying unit 110 may be configured to display a visual user interface of the planning unit 108.

Fig. 1B shows a laser, which may be implemented in the system shown in Fig. 1A.

The laser includes a laser power unit 120, a beam expander 122, a shutter 124, an X-Y-Z-Galvanometer and a relay lens 126. These components may be included in the laser base unit 102 shown in Fig. 1A.

The patient interface 106 shown in Fig. 1A comprises a relay lens 138 and a focusing lens 140 mounted on a piezoelectric stage, which may be adjusted in X, Y, and Z direction.

The pulsed laser beam generated in the laser base unit 102 is then guided to the patient interface 106 via mirrors 130, 132, 134 and 136, which may be located inside the support arm 116. The patient interface including the focusing lens 140 is configured to emit the laser pulses to the patient's eye, and, in particular, to set the laser focus to the endothelium and/or Descemet's membrane. From the patient interface, the laser beam is guided to the patient's eye 142, which is fixed and/or flattened by the fixation unit 104.

In order o allow for an improved and precise optical tissue breakdown without larger air bubble generation, the spot size may preferably be minimized while lowering the pulse energy (preferably below 100 pJ). Therefore, the large focusing lens is preferably kept on the mentioned piezoelectric stage within a few centimetres above the mounted eyeball. On-line alignment of the focusing lens may preferably be provided according to the automatically or manually selected cutting patterns. Furthermore, to reduce optical aberrations and thus reduce spot size, the laser beam may preferably be aligned precisely perpendicular to the human's cornea. In this way, cutting patterns with a resolution of < 5 µm in X-, Y- and Z-direction may be achieved. For example, using an NA of 0.3 in a system with low optical aberrations would lead to a spot size below 4 µm in diameter.

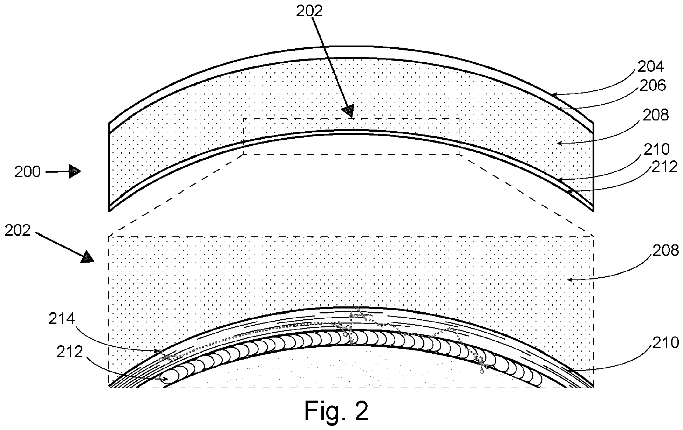

Fig. 2 shows a portion of an eye's cornea 200. In the anterior-posterior direction, the cornea 200 comprises the corneal epithelium 204, the Bowman membrane 206, the corneal stroma 208, the Descemet's membrane 210. Section 202 is an enlargement of the portion of the cornea 200 comprising the corneal stroma 208, the Descemet's membrane 210 and the corneal endothelium comprising corneal endothelial cells 212.

Reference numeral 214 denotes an exemplary cutting pattern, which may be provided with the system described herein. As can be seen, the cutting pattern 214 comprises a first portion that is located in the Descemet's membrane 210 and a second, varying cutting pattern portion, which has a varying depth and extends partly in the Descemet's membrane and partly in the endothelial cells 212.

## Claims

1. System (100) for *in vivo* treatment of a patient's eye's endothelium (212) and/or Descemet's membrane (210) by laser therapy, comprising:
a laser unit comprising a short-pulse laser (102); and
a fixation unit (104) for fixing the patient's eye;
wherein the laser unit is configured to set the laser focus to the endothelium (212) and/or Descemet's membrane (210) and wherein the laser is adapted to generate tissue effects having an accuracy of 5 µm or less in laser-beam direction in the endothelium (212) and/or Descemet's membrane (210).

2. System (100) according to any one of the preceding claims,
wherein the laser (102) has a wavelength above 800 nm; and/or
wherein the pulse duration of the laser (102) is shorter than one microsecond (1 µs); and/or
wherein the numerical aperture (NA) of the laser (102) is greater than 0.3

3. System (100) according to any one of the preceding claims, wherein the laser (102) has a cutting accuracy of 5 µm or less, preferably of 4 µm or less, more preferably of 3 µm or less, in every direction (X, Y and Z) in a distance of more than 200 µm or preferably a distance of more than 300 µm from the anterior corneal surface.

4. System (100) according to any one of the preceding claims, further comprising:
an imaging unit (100) for capturing an image of the patient's eye's cornea (200), preferably of the posterior part of the cornea (120), more preferably of the Descemet's membrane (210) and endothelium (212),
wherein the imaging unit's resolution is 5 µm or less in every dimension.

5. System (100) according to claim 1,
wherein the imaging unit comprises an optical coherence tomography (OCT) unit.

6. System (100) according to claim 5,
wherein the OCT unit is a polarization sensitive OCT unit, a spectral domain OCT unit, a Fourier domain OCT unit and/or a swept source OCT unit; and/or
wherein the axial resolution and the lateral resolution in corneal tissue of the OCT unit is 5 µm or less, preferably 4 µm or less, more preferably 3 µm or less; and/or
wherein the OCT unit has a power of 20mW or less; and/or
wherein the OCT unit has a central wavelength in the range of 600 nm to 1400 nm.

7. System (100) according to any one of claims 4 to 6,
wherein the imaging system comprises one or more of a confocal microscopy unit, a Scheimpflug camera, a light microscope, an ultrasound biomicroscope, a specular microscopy unit

8. System (100) according to any one of claims 4 to 7,
wherein the imaging system is configured to capture at least one image of the patient's cornea (200) before and/or during the treatment of the patient's eye's endothelium (212) and/or Descemet's membrane (210) by laser therapy;
wherein the at least one image preferably comprises at least a portion of diseased tissue of the endothelium (212) and/or Descemet's membrane (210);
wherein, the at least one image preferably comprises at least a portion of the tissue effects generated by the laser (102);
wherein the imaging system is preferably configured to visualize the at least one image.

9. System (100) according to any one of the preceding claims, further comprising:
an aiming unit configured to aim the laser focus to a predetermined portion of the patient's endothelium (212) or Descemet's membrane (210).

10. System (100) according to any one of the preceding claims, further comprising:
a planning unit (108) configured to assist a user in planning and/or executing the *in vivo* treatment of the patient's eye's endothelium (212) and/or Descemet's membrane (210) with the laser unit, preferably on the basis of the at least one image captured by the imaging system of claims 4 to 8.

11. System (100) according to claim 10,
wherein the planning unit (108) is configured to assist a user in planning the tissue effects in the patient's cornea (200) with the laser unit (102); and/or
wherein the planning unit (108) is configured to automatically detect diseased tissue and/or anatomical landmarks of the patient's cornea (200); and/or
wherein the planning unit (108) is configured to determine the tissue effects in the patient's cornea (200) and to represent the tissue effects in the captured image; and/or
wherein the planning unit (108) is configured to assist a user in three-dimensional planning of the treatment.

12. System (100) according to any one of the preceding claims,
wherein the fixation unit (104) is configured to fix the patient's eyeball, to center the cornea (200), and/or to flatten the cornea (200); and/or
wherein the fixation unit (104) comprises a mechanism for automatically tracking the eyeball during treatment.

13. Method of *in vivo* treatment of a patient's eye's endothelium (212) and/or Descemet's membrane (210) by laser therapy, preferably with a system (100) according to any one of the preceding claims, the method comprising the steps:
fixing and preferably flattening the patient's eye;
generating tissue effects in the patient's eye's endothelium (212) and/or Descemet's membrane (210) with a laser (102).

14. Method according to claim 13, further comprising the step:
capturing an image of the patient's eye's cornea (200).

15. Method according to claim 14, further comprising the step:
planning the tissue effects in the patient's eye's endothelium (212) and/or Descemet's membrane (210) based on the captured image.

16. Use of a laser (102) for *in vivo* treatment of a patient's eye's endothelium (212) and/or Descemet's membrane (210).
